# EUROPÄISCHE PATENTSCHRIFT

(11) **EP 1 529 524 B1**
(45) Veröffentlichungstag und Bekanntmachung des Hinweises auf die Patenterteilung: **01.02.2006**
(21) Anmeldenummer: 03025259.7
(22) Anmeldetag: 06.11.2003
(51) Int. Cl.: A61K 9/48, A61K 31/59, A61K 31/07, A61K 31/355, A61K 31/122, A61K 31/4415

(54) **Gelatinekapsel mit fettlöslichen Vitaminen und essentiellen Fettsäuren**
Softgelatine capsule comprising fat-soluble vitamins and essential fatty acids
Gélule comprenant des vitamines liposolubles et des acides gras essentiels

(43) Veröffentlichungstag der Anmeldung: 11.05.2005
(73) Patentinhaber: Meduna Arzneimittel GmbH, 30916 Isernhagen (DE)
(72) Erfinder: Frauendorfer, Friedel, 30938 Burgwedel (DE)
(74) Vertreter: Schildberg, Peter

(56) Entgegenhaltungen:
- WO-A-01/01797
- DE-A- 4 304 394
- DE-A- 4 405 545
- US-A1- 2003 054 083

## Beschreibung

Die vorliegende Erfindung betrifft eine orale Darreichungsform für Nahrungs- sowie Nahrungsergänzungsmittel und Diätetika, die sich insbesondere zum Einsatz bei der Behandlung von Fettabsorptionsstörungen und deren Folgeerkrankungen eignet.

Die Vitamine A, D, E und K benötigen Fett, um vom menschlichen Körper absorbiert werden zu können. Bei Fettabsorptionsstörungen tritt daher ein Mangel an essentiellen Fettsäuren und an fettlöslichen Vitaminen A, D, E und K im Körper auf. Dieser Mangel wird bei Fettabsorptionsstörungen unter anderem verantwortlich gemacht für das Auftreten von Durchblutungsstörungen, Mikroangiopathien, Osteoporose, Anämie und allgemeinen Entzündungserscheinungen.

Insbesondere bei chronisch-entzündlichen Darmerkrankungen, vor allem bei M. Crohn, wurden klinisch bedeutsame Störungen der Resorption fettlöslicher Vitamine nachgewiesen. Ein Vitamin-A-Mangel (Sehstörungen, Schleimhautschäden) wurde bei 11% der Patienten und ein Vitamin-D-Mangel (Abnahme von Knochengewebe) bei 75% der Patienten festgestellt (Adler, G. - Morbus Crohn und Colitis ulcerosa, Springer Verlag 1996). Die Deutsche Gesellschaft für Verdauungs- und Stoffwechselkrankheiten empfiehlt in einer Leitlinie von 2003 die generelle Vitamin D-Supplementierung bei Patienten mit M. Crohn, die länger als 6 Monate mit Steroiden behandelt wurden.

Bei dem Produkt Modulen ® IBD der Firma Nestle ist ein diätetisches Lebensmittel für besondere medizinische Zwecke (bilanzierte Diät) entwickelt worden. Das Produkt ist zur diätetischen Behandlung von Morbus Crohn bei Kindern ab 5 Jahren, Heranwachsenden und Erwachsenen geeignet. Im Rahmen einer Ernährungstherapie ist es möglich, sowohl die Symptome im akuten Schub (kolikartige Schmerzen, Fieber, Übelkeit und Diarrhoe) zu lindern als auch den mit dem Krankheitsbild einhergehenden, unzureichenden Ernährungszustand (z. B. nach Darmoperation) zu verbessern. Das Produkt enthält gesättigte Fettsäuren, einfach und mehrfach ungesättigte Fettsäuren, Spurenelemente, Vitamine und Mineralstoffe und liegt als wasserlösliches Pulver vor. Das Pulver ist in erster Linie zur Verwendung als Trinknahrung konzipiert, kann aber auch über eine Ernährungssonde eingesetzt werden.

WO 01/01797 beschreibt eine dünndarmgesteuerte, xylosebeschichtete Weichgelatinekapsel, die alpha-Linolensäure in Form von Perillaöl enthält. Es wird jedoch nicht erwähnt, dass fettlösliche Vitamine in die Kapsel eingearbeitet werden können, um im Körper besser resorbiert zu werden.

Aus EP 1 091 659 B1, die ebenfalls auf die Anmelderin zurückgeht, ist eine orale Darreichungsform bekannt, die mehrfach ungesättigte Fettsäuren in einer xylosegehärteten Gelatinekapsel mit einer verzögerten Kapselöffnungszeit besitzt. Es ist bekannt, diese Gelatinekapsel bei Fettstoffwechselstörung und/oder Darmentzündung, wie beispielsweise Morbus Crohn oder Colitis ulcerosa, anzuwenden.

Der Erfindung liegt die Aufgabe zugrunde, eine feste orale Darreichungsform zu schaffen, die sich insbesondere zum Einsatz bei Fettabsorptionsstörungen und deren Folgeerkrankungen eignet.

Erfindungsgemäß wird die Aufgabe durch eine orale Darreichungsform mit den Merkmalen aus Anspruch 1 gelöst. Vorteilhafte Ausgestaltungen bilden den Gegenstand der Unteransprüche.

Bei der oralen Darreichungsform gemäß Anspruch 1 werden in Fettsäuren eingearbeitete Vitamine A, D, E, und K in einer Gelatinekapsel bereitgestellt. Die Fettsäuren bestehen aus einem Gemisch von Fettsäuren mit Alpha-Linolensäure (ALA), Docosahexaensäure (DHA) und mittelkettigen Triglyceriden (MTC). Überraschenderweise hat sich herausgestellt, dass das Gemisch aus Fettsäuren mit den Vitaminen bei dieser oralen Darreichung zu einer besonders hohen Absorption und einem hohen Wirkspiegel in Blut und Gewebe führt. Die erfindungsgemäße Fettsäurenzusammensetzung, insbesondere in Verbindung mit Perillaöl als bevorzugter Alpha-Linolensäurelieferant wurde bisher nicht beschrieben und stellt einen optimalen Lösungsvermittler, lipophilen Träger und Transportmedium für die fettlöslichen Vitamine dar.

Bei einer bevorzugten Weiterbildung der oralen Darreichungsform wird dem Gemisch zusätzlich Vitamin B, insbesondere Vitamin B6 zugesetzt. Es hat sich in einer experimentellen Studie (Effects of vitamin B6 on (n-3) polyunsaturated fatty acid metabolism / Tsuge, H.. - 2000) herausgestellt, dass das zusätzliche Einmischen von Vitamin B6 die Eigenwirkung von Perillaöl unterstützt.

Es wurde ferner festgestellt, dass für die orale Darreichungsform als Gelatinekapsel besonders eine xylosebeschichtete Weichgelatinekapsel, wie beispielsweise in EP 1 091 659 B1 beschrieben, geeignet ist, die den Kapselinhalt nach der Einnahme verzögert freigibt. Die Verzögerungs- und Zerfallszeit der Kapsel wird dabei so eingestellt, dass das Gemisch aus Fettsäuren und Vitaminen bevorzugt erst im Dünndarm, dem Ort der wesentlichen Resorption, freigesetzt wird.

Die erfindungsgemäße orale Darreichungsform dient zur Behandlung von Vitamin- und Fettsäure-Mangelerscheinungen, wie sie bei zystischer Fibrose, Morbus Crohn und bei chologener Diarrhoe auftreten. Diese Krankheitsbilder zeichnen sich durch eine unzureichende Fettverdauung und ein Absorptionsproblem für wesentliche Nahrungsbausteine aus, insbesondere durch einen Mangel an lebenswichtigen Stoffen, wie den fettlöslichen Vitaminen A, D, E und K sowie essentiellen Fettsäuren.

Zur Behandlung dieser Mangelerscheinungen wird erfindungsgemäß eine orale, feste, exakt dosierbare, dünndarmgesteuerte Darreichungsform vorgeschlagen, die eine ausreichende Absorption fettlöslicher Vitamine zusammen mit essentiellen Fettsäuren erlaubt.

Eine bisher unerreicht hohe Absorption der Wirkstoffmengen wird also sowohl durch die dünndarmgesteuerte Kapsel, als auch durch die Einmischung der fettlöslichen Vitamine in das vorgeschlagene Fettsäuregemisch erreicht.

Die oben angesprochene Sondennahrung "Modulen" dient im Gegensatz dazu der künstlichen Ernährung, wenn normale ballaststoffreiche Ernährung nicht möglich ist. Die Ansprüche einer hohen Absorption von therapeutischen Wirkstoffmengen, exakter Dosierung und bequemer Einnahme kann die Sondennahrung nicht erfüllen.

### Beispiel:

Die fettlöslichen Vitamine A, D, E und K werden in ein Gemisch aus Fettsäuren, deren Hauptbestandteil Perillaöl ist, eingearbeitet. Dabei fungiert das Fettsäuregemisch gleichzeitig als eigenständiger Wirkstoff, als Lösungsvermittler, sowie als lipophiler Träger für die Vitamine. Die Absorption fettlöslicher Vitamine erfolgt, wenn diese in Lipoproteine eingeschlossen sind. Dabei sichert das zusätzliche Einmischen von Vitamin B6 die gesundheitsfördernden Eigenschaften und nutritiven Effekte bestimmter Fettsäuren, wie Alpha-Linolensäure aus Perillaöl.

Um für das Krankheitsgeschehen eine optimale, therapeutische Fettsäurebilanz wie auch -absorption der Vitamine A, D, E und K zu erzielen, wurde folgendes Fettgemisch eingesetzt:

| | |
|---|---|
| 33 bis 65% | Alpha-Linolensäure (ALA), |
| 2,5 bis 30% | Docosahexaensäure (DHA) und |
| 10 bis 35% | mittelkettige Triglyceride (MCT). |

Für eine Tagesration sind 1 bis 3 Gramm sinnvoll.

Diese Kombination aus Fettsäuren haben für die genannte Tagesration eine überraschend intensive Absorption der Vitamine A, D, E und K ergeben. Zum Vergleich wurden nicht xylosebeschichtete Vitaminkapseln ohne die oben genannten Fettsäuren, mit handelsüblichen Ölen gegeben, wobei das erfindungsgemäße Fettsäurengemisch zu einer mehr als 33% höheren Absorption der Vitamine geführt hat.

Die Anwendung in der dünndarmgesteuerten Kapsel mit Xylosebeschichtung erhöhte die Absorptionsmenge der Vitamine weiter auf über 50% gegenüber dem Vergleichsprodukt (handelsübliche Gelatinekapsel ohne Xylosebeschichtung mit gleicher Vitaminzusammensetzung in Sojaöl). Es wird angenommen, dass eine intensive Anbindung der Vitamine an die Fettsäuren mit der hohen Wirkstoffbereitstellung im Dünndarm durch die magensaftresistente Kapsel, sowie die spezielle Fettsäurezusammensetzung der erfindungsgemäßen Kapsel überhaupt erst eine ausreichende Resorption der fettlöslichen Vitamine durch die entzündete und teilweise zerstörte Schleimhaut bei diesen Patienten ermöglicht.

Die Zugabe von reinem DHA erhöht die antientzündliche Eigenschaft der ALA in Perillaöl und MCT stellt sofort Energie bereit, da MCT keine Gallensalze benötigt, um die Fettabsorption sofort in Gang zu setzen.

Die Vitaminanteile für den Tagesbedarf orientieren sich an Alter und Gewicht des Patienten und am Schweregrad der Absorptionsstörungen.

Die folgende Vitaminzusammensetzung pro Kapsel erscheint sinnvoll:

| | | | | | |
|---|---|---|---|---|---|
| Vitamin A | 0,5 | mg | bis | 3,0 | mg (Retinol-Äquivalent) |
| Vitamin D | 1,50 | µg | bis | 5,00 | µg |
| Vitamin E | 20,0 | mg | bis | 60,0 | mg |
| Vitamin K | 15,0 | µg | bis | 100,0 | µg |

Als wasserlösliches Vitamin wurde Vitamin B6 in einer Menge von 0,5 bis 3,0 mg zugegeben.

Folgende Wirkstoffzusammensetzung der magensaftresistenten Kapseln werden beispielsweise ausgeführt:

| **Kapsel A** | | | **Kapsel B forte** | | |
|---|---|---|---|---|---|
| Alpha-Linolensäure aus Perillaöl (215 mg) | | | (430,00 mg) | | |
| | 125,00 | Mg | | 250,00 | mg |
| DHA aus Fischöl | | | | | |
| Konzentrat | 50,00 | Mg | | 100,00 | mg |
| MCT | 75,00 | Mg | | 150,00 | mg |
| Vitamin E | 20,00 | Mg | | 60,00 | mg |
| Vitamin A | 0,50 | Mg | | 2,00 | mg |
| Vitamin D | 1,50 | µg | | 3,00 | µg |
| Vitamin K | 15,00 | µg | | 30,00 | µg |
| Vitamin B6 | 0,50 | Mg | | 2,00 | mg |

Tagesdosis 3 x 1 bis 3 x 2 Kapseln, je nach Körpergewicht und Substitutionsbedarf.

Die in Perillaöl unter Zufügung der Fettsäuren DHA und MCT eingearbeiteten, fettlöslichen Vitamine A, D, E und K werden in eine xylosebeschichtete Weichgelatinekapsel eingebracht. Diese Kapseln besitzen durch die Xylosehärtung Magensaftresistenz und weisen eine gesteuerte Wirkstofffreisetzung auf, so dass die Wirkstoffe den Magen passieren und erst im Dünndarm zur Absorption freigesetzt werden.

## Patentansprüche

1. Orale Darreichungsform für Nahrungs- sowie Nahrungsergänzungsmittel und Diätetika, insbesondere zum Einsatz bei Fettabsorptionsstörungen, die eine Gelatinekapsel mit in einem Fettsäuregemisch gelösten Vitaminen A, D, E und K aufweist, wobei das Fettsäuregemisch Alpha-Linolensäure (ALA), Docosahexaensäure (DHA) und mittelkettige Triglyceride (MCT) aufweist.

2. Orale Darreichungsform nach Anspruch 1, **dadurch gekennzeichnet, dass** das Fettsäuregemisch Perillaöl als Lieferant von Alpha-Linolensäure (ALA) aufweist.

3. Orale Darreichungsform nach Anspruch 1 oder 2, **dadurch gekennzeichnet, dass** in der Gelatinekapsel zusätzlich Vitamin B enthalten ist, insbesondere Vitamin B6.

4. Orale Darreichungsform nach einem der Ansprüche 1 bis 3, **dadurch gekennzeichnet, dass** die Gelatinekapsel eine xylosebeschichtete Weichgelatinekapsel ist, die ihren Kapselinhalt nach der Einnahme verzögert freigibt.

## Claims

1. Oral pharmaceutical form for foodstuffs as well as dietary supplements, in particular for use in fat-absorption disorders, which have a gelatin capsule with vitamins A, D, E and K dissolved in a fatty-acid mixture, whereby the fatty-acid mixture has alpha-linolenic acid (ALA), docosahexaenoic acid (DHA)and medium-chain triglyceride (MCT).

2. Oral pharmaceutical form according to claim 1, **characterized in that** the fatty-acid mixture has perilla oil as the supplier of alpha-linolenic acid (ALA).

3. Oral pharmaceutical form according to claim 1 or 2, **characterized in that** vitamin B, in particular vitamin B6, is contained in the gelatin capsule.

4. Oral pharmaceutical form according to one of claims 1 through 3, **characterized in that** the gelatin capsule is a xylose-coated soft-gelatin capsule, which delay-releases it capsule contents after ingestion.

## Revendications

1. Forme de présentation orale pour produits alimentaires et compléments alimentaires et produits diététiques, en particulier pour la mise en oeuvre dans le cas de problèmes d'absorption des graisses, qui présente une gélule avec des vitamines A, D, E et K dissoutes dans un mélange d'acides gras, le mélange d'acides gras présentant de l'acide alpha-linolénique (ALA), de l'acide docosahexaénoïque (DHA) et des triglycérides à chaîne moyenne (MCT).

2. Forme de présentation orale selon la revendication 1, **caractérisée en ce que** le mélange d'acides gras présente de l'huile de perilla en tant que source d'acide alpha-linolénique (ALA).

3. Forme de présentation orale selon la revendication 1 ou 2, **caractérisée en ce que** de la vitamine B, en particulier de la vitamine B6, est contenue à titre supplémentaire dans la gélule.

4. Forme de présentation orale selon une des revendications 1 à 3, **caractérisée en ce que** la gélule est une gélule tendre revêtue de xylose qui libère son contenu de gélule de façon temporisée après la prise.
